# EUROPEAN PATENT SPECIFICATION

(11) **EP 2 856 922 B1**
(45) Date of publication and mention of the grant of the patent: **10.10.2018**
(21) Application number: 13793831.2
(22) Date of filing: 16.04.2013
(51) Int. Cl.: A61B 1/00, A61B 1/04, A61B 1/05, A61B 5/107, G02B 23/24, G02B 13/00, G06T 7/571, G02B 27/22, H04N 13/00

(54) **STEREOSCOPIC ENDOSCOPE DEVICE**
STEREOSKOPISCHE ENDOSKOPVORRICHTUNG
DISPOSITIF D'ENDOSCOPE STÉRÉOSCOPIQUE

(30) Priority: 24.05.2012 JP 2012118754
(43) Date of publication of application: 08.04.2015
(73) Proprietor: Olympus Corporation, Tokyo 192-8507 (JP)
(72) Inventor: IKEDA, Hiromu, Hachioji-shi, Tokyo 192-8507 (JP)
(74) Representative: Schicker, Silvia
(86) International application number: PCT/JP2013/061304
(87) International publication number: WO 2013/175899

(56) References cited:
- WO-A1-2011/158498
- WO-A1-2012/075631
- WO-A1-2013/046902
- JP-A- 2001 103 513
- JP-A- 2001 108 916
- JP-A- 2001 108 916
- JP-A- 2004 313 523
- JP-A- 2004 337 379
- JP-A- 2010 092 283
- JP-A- 2010 092 283
- JP-A- 2011 211 739
- JP-A- 2011 211 739
- JP-A- 2012 022 147
- JP-B1- 2 963 990
- JP-B1- 2 963 990
- US-A1- 2004 125 228
- US-A1- 2009 259 098
- US-A1- 2010 245 550

## Description

### {Technical Field}

The present invention relates to a stereoscopic endoscope device.

### {Background Art}

There are known conventional cameras in which a single objective lens is driven in an optical-axis direction, thereby capturing a subject multiple times at different focal positions, a plurality of acquired images are used to calculate distance distribution information about a distance from the objective lens to the subject, and the calculated distance distribution information is used to generate parallax images of the subject (see PTL 1, for example). The parallax images are a pair of viewpoint-images of the subject when observed from two viewpoints corresponding to the right and left eyes of an observer. A stereoscopic image of the subject can be created from the parallax images.

PTL 2 discloses an endoscopic apparatus including a light-generating mechanism for generating at least one modulated measuring beam, a light-transmitting mechanism for conducting the measuring beam onto an area to be observed, where the light-transmitting mechanism includes an illuminating lens, in addition to a light-imaging mechanism for imaging a signal beam from the area to be observed at least onto a phase-sensitive image sensor, which includes a number of pixels, where the light-imaging mechanism includes an imaging lens; at least one additional image sensor is provided and the light-imaging mechanism includes a beam-splitter and/or an optical switch, where at least one adaptive lens is provided for adapting the fields of vision of the image sensors.

PTL 3 discloses a stereoscopic image creation method for generating a stereo image including a first and second parallax image, allowing the stereoscopic vision, wherein a two-dimensional original image is acquired, the depth direction of each pixel is obtained on the basis of the luminance information of each pixel of the original image and a pseudo three-dimensional image information including coordinates of lateral direction, longitudinal direction and depth direction of the image, is generated for each pixel of the original image, and the original image is three-dimensionally rotated rightward and leftward by prescribed angles by means of using the pseudo three-dimensional image information, to generate the first, respectively second image.

PTL 4 discloses distance measuring device provided with an opening mask as light-passing means, a lens system converging lights entered from the opening mask and CCDs, the optical configurations of which are each other different, and a calculation part, that compares and analyzes the blurring contained in the obtained images to determine a focusing position on the basis of the blurring quantity.

PTL 5 discloses an image capture device that generates a distance map of a photographic subject from a photographic image, with high precision, wherein three images are photographed with a sensor drive unit and a sensor drive controller unit, which move an image sensor in the optical axis direction: photographic images that are respectively focused on the near-end side and the far-end side of the photographic subject, and an image that is photographed while sweeping the image sensor from the near-end side to the far-end side; an omnifocal image is generated by an omnifocal image generator unit from the sweep image; a blur quantity in each component region of the image and the image is computed by a blur quantity computation unit, carrying out a deconvolution process with an image of a region corresponding to the omnifocal image and the distance from the device to the photographic subject in each image region, i.e., a distance map, is generated by a distance map generator unit from the blur quantity of the regions corresponding to the near-end image and the far-end image and optical coefficients of the image capture device, including the focal length of the lens.

A further stereoscopic vision image preparation device, output device and method are known from PTL 6.

PTL 7 discloses a stereoscopic device according to the preamble of claim 1.

According to PTL 1, because such a pair of images can be generated by using a single objective lens, the configuration is reduced in size compared with a camera having two objective
lenses corresponding to right and left eyes and therefore can be suitably applied to an endoscope.

### {Citation List}

### {Patent Literature}

{PTL 1} Japanese Unexamined Patent Application, Publication No. Hei 5-7373;
{PTL 2} American Patent Application, Publication No. US2009259098 (A1);
{PTL 3} Japanese Patent Application, Publication No. JP2010092283 (A);
{PTL 4} Japanese Patent Application, Publication No. JPH11337313 (A);
{PTL 5} International Patent Application, Publication No. WO2011158498 (A1);
{PTL 6} Japanese Patent Application, Publication No. JP2011211739 (A);
{PTL 7} American Patent Application, Publication No. US2004125228 (A1).

### {Summary of Invention}

### {Technical Problem}

However, in the case of PTL 1, in order to acquire the parallax images, the subject needs to be captured multiple times while driving the objective lens, thus taking a relatively long time. Therefore, there is a problem in that it is difficult to observe a stereoscopic image of the subject in real-time as a moving image.

The present invention has been made in view of the above-described circumstances, and an object thereof is to provide a stereoscopic endoscope device capable of generating a stereoscopic moving image of the subject in real-time.

### {Solution to Problem}

In order to achieve the above-described object, the present invention provides the following solutions.

The present invention provides a stereoscopic endoscope
device including the features of claim 1. Preferred embodiments are defined by the dependent claims. Any example or embodiment which does not fall under the scope of the claims is not part of the invention.

According to the present invention, after light from the subject, collected by the objective lens, is split into two or more beams by the light splitting section, the two or more beams are given optical path lengths different from each other by the focal-position adjusting sections and are then captured by the image-capturing devices. Therefore, 2D images acquired by the image-capturing devices are images of an identical
field of view captured at different focal positions.

The calculation section calculates the distribution of object distances of the subject from such a plurality of 2D images whose focal positions are different, and the parallax-image generating section generates, through calculation, a plurality of viewpoint images based on the calculated distribution of object distances.

In this case, the plurality of viewpoint images, which are the base of parallax images, are captured at the same time, and parallax images can be generated at sufficiently short intervals. Thus, a stereoscopic moving image of the subject can be generated in real-time.

In the above-described invention, the parallax-image generating section may set a space between the plurality of viewpoints to a distance smaller than a diameter of the objective lens.

In the case in which parallax images are generated, through calculation, from a plurality of 2D images acquired by using a single objective lens, the distance (base-line length) between viewpoints can be set regardless of the diameter of the objective lens. In a stereoscopic image created from the parallax images, a sense of depth is emphasized as the base-line length is increased. Therefore, it is possible to reproduce, in the stereoscopic image, such a sense of depth of the subject that could not be reproduced in a configuration in which two viewpoint images are acquired by using two objective lenses.

In the above-described invention, a display unit that creates a stereoscopic image from the plurality of viewpoint-images generated by the parallax-image generating section and displays the stereoscopic image may be included, and the parallax-image generating section may generate parallax images in which the object distance and a depthwise reproduction distance reproduced in the stereoscopic image displayed in the display unit have a substantially linear relationship.

By doing so, in a stereoscopic image, a depthwise distance of the subject is accurately reproduced. Therefore, the observer can accurately understand the depthwise position of the subject from the stereoscopic image.

In the above-described invention, a display unit that creates a stereoscopic image from the plurality of viewpoint-images generated by the parallax-image generating section and displays the stereoscopic image may be included, and the parallax-image generating section may generate parallax images in which the object distance and a depthwise reproduction distance reproduced in the stereoscopic image displayed in the display
unit have a non-linear relationship in which a variation in the reproduction distance with respect to a variation in the object distance is convex upward.

By doing so, in a stereoscopic image, a depthwise distance of the subject is reproduced in a compressed manner. Therefore, when a subject having depth is observed in the stereoscopic image, a feeling of eye fatigue given to the observer from the stereoscopic image can be reduced.

In the above-described invention, the parallax-image generating section may set a space between the plurality of viewpoints to 5 mm or less.

By doing so, even when a subject to be captured is small, a sense of depth of the subject reproduced in a stereoscopic image can be made appropriate.

In the above-described invention, the parallax-image generating section may set a space between the plurality of viewpoints to a plurality of distances and generate a plurality of parallax images.

By doing so, an identical subject can be observed in a plurality of stereoscopic images having different senses of depth.

In the above-described invention, the light splitting section may output the two or more split beams of the light almost parallel to each other; and an imaging plane of the image-capturing devices may be divided into two or more areas, and the two or more beams of the light output from the light splitting section may be captured in different two or more areas.

By doing so, a common image-capturing device is used, thus making it possible to achieve a simpler configuration.

In the above-described invention, the focal-position adjusting sections may include two prisms that are joined together and are disposed such that a joint surface of the two prisms intersects with an optical axis of the objective lens; and the light splitting section may include a beam splitter that is provided on the joint surface and that allows a part of light entering one of the prisms from the objective lens to be transmitted through the other prism and the other part of the light to be deflected in a direction intersecting the optical axis.

By doing so, it is possible to form the light splitting section and the focal-position adjusting sections into an integral structure, thus simplifying the configuration.

In the above-described invention, the light splitting section may have an outer diameter smaller than outer
diameters of the image-capturing devices.

By doing so, the configuration can be further reduced in size.

In the above-described invention, the parallax-image generating section may generate parallax images such that an intersection of virtual lines of sight for observing the subject from viewpoints is located between focal positions of the two or more beams of the light to be captured by the image-capturing devices.

By doing so, it is possible to acquire an image at a position close to the focal position in the central observation distance of the image, thereby providing the image with a high degree of sharpness.

In the above-described invention, each of the image-capturing devices may comprise an imaging-state detecting section that detects an imaging state of the light to be captured, by a phase difference method.

By doing so, an imaging state of the light captured by the image-capturing device can be detected with a simple configuration.

### {Advantageous Effects of Invention}

According to the present invention, an advantageous effect is afforded in that a stereoscopic moving image of the subject can be generated in real-time.

### {Brief Description of Drawings}

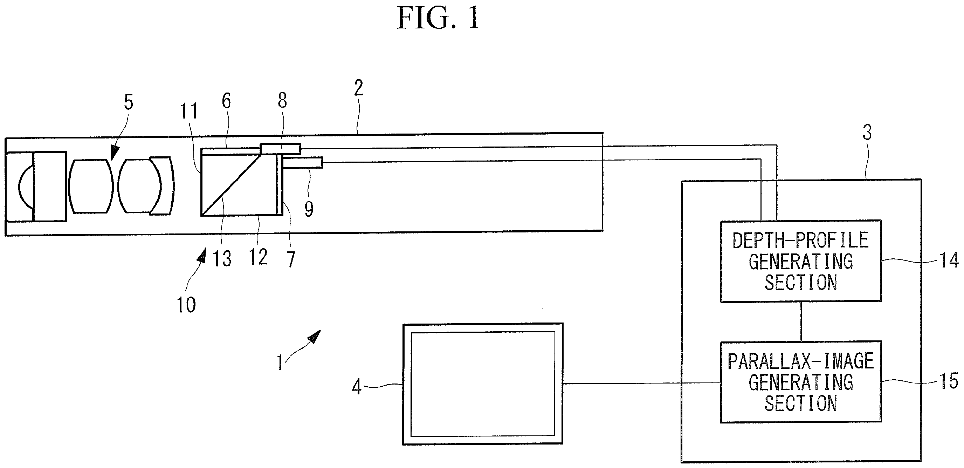

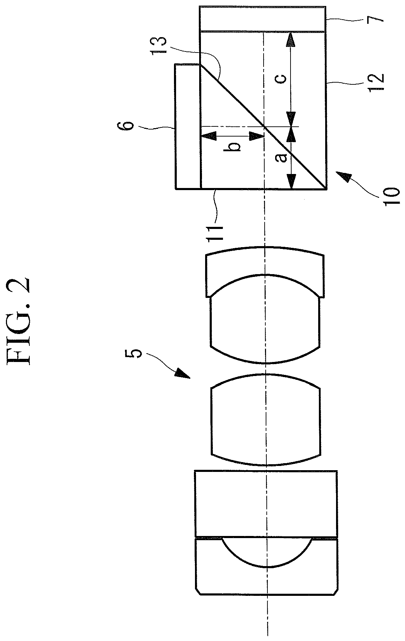

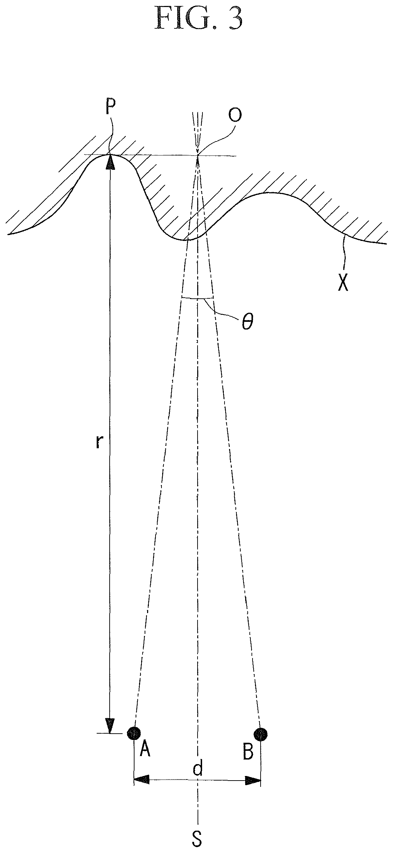

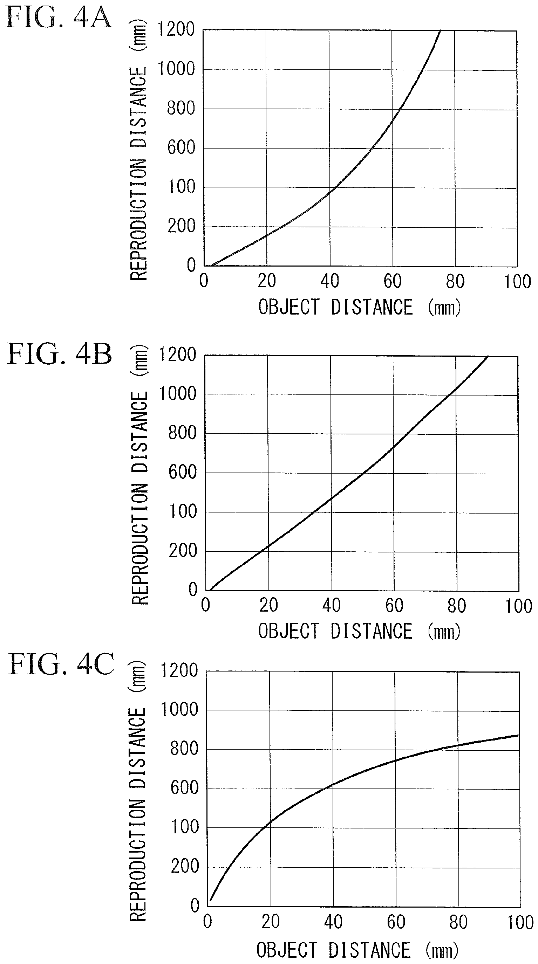

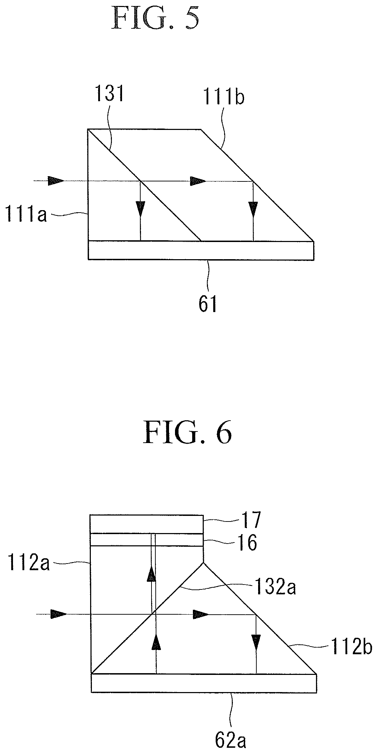

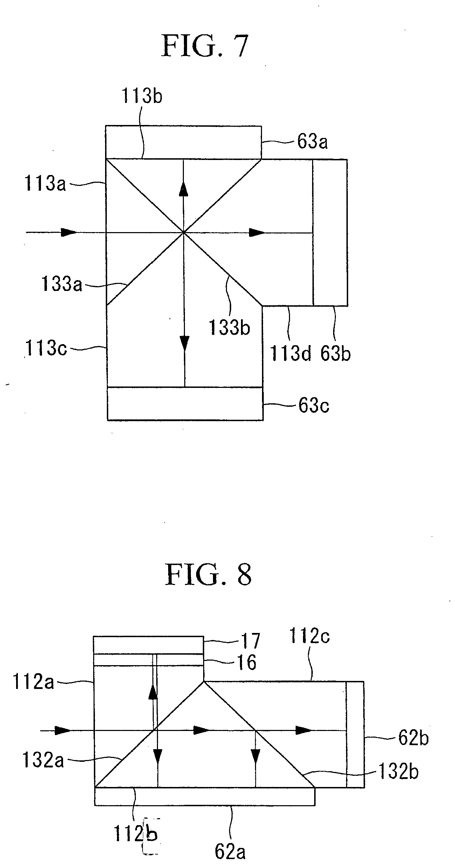

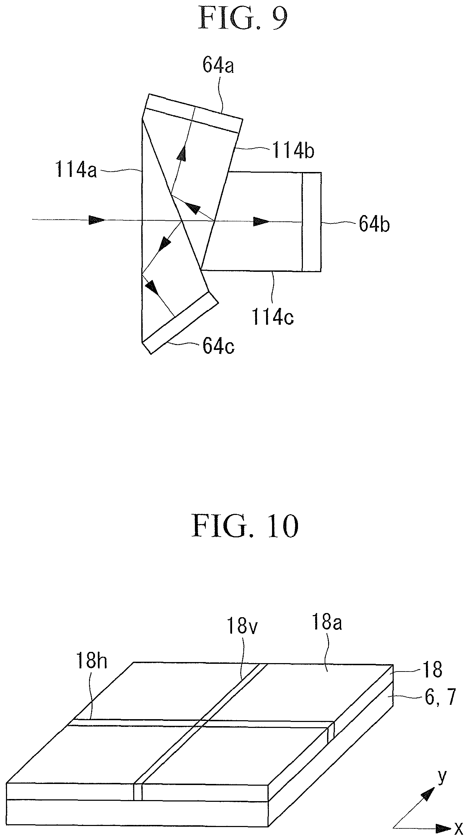

{Fig. 1} Fig. 1 is a diagram showing the overall configuration of a stereoscopic endoscope device according to one embodiment of the present invention.
{Fig. 2} Fig. 2 is a diagram showing, in enlarged form, an objective lens and a prism-type beam splitter included in the stereoscopic endoscope device shown in Fig. 1.
{Fig. 3} Fig. 3 is a diagram for explaining parameters set by a parallax-image generating section shown in Fig. 1 to generate parallax images.
{Fig. 4A} Fig. 4A is a graph showing the relationship between an object distance and a reproduction distance when a base-line length is set to 5 mm.
{Fig. 4B} Fig. 4B is a graph showing the relationship between the object distance and the reproduction distance when the base-line length is set to 3 mm.
{Fig. 4C} Fig. 4C is a graph showing the relationship between the object distance and the reproduction distance when the base-line length is set to 1 mm.
{Fig. 5} Fig. 5 is a diagram showing a partial configuration of a modification of the stereoscopic endoscope device shown in Fig. 1.
{Fig. 6} Fig. 6 is a diagram showing a partial configuration of another modification of the stereoscopic endoscope device shown in Fig. 1.
{Fig. 7} Fig. 7 is a diagram showing a partial configuration of still another modification of the stereoscopic endoscope device shown in Fig. 1.
{Fig. 8} Fig. 8 is a diagram showing a partial configuration of still another modification of the stereoscopic endoscope device shown in Fig. 1.
{Fig. 9} Fig. 9 is a diagram showing a partial configuration of still another modification of the stereoscopic endoscope device shown in Fig. 1.
{Fig. 10} Fig. 10 is a diagram showing a partial configuration of still another modification of the stereoscopic endoscope device shown in Fig. 1.

### {Description of Embodiment}

A stereoscopic endoscope device 1 according to one embodiment of the present invention will be described below with reference to the drawings.

As shown in Fig. 1, the stereoscopic endoscope device 1 of this embodiment includes an endoscope main body (hereinafter, also referred to as a main body) 2 that captures a subject at different focal positions, thereby acquiring two 2D images, an image processing unit 3 that receives the two 2D images from the main body 2 and generates parallax images from the 2D images, and a stereoscopic image display unit (display unit) 4 that creates a stereoscopic image from the parallax images generated by the image processing unit 3 and displays the stereoscopic image.

The main body 2 includes a single objective lens 5 that collects light from the subject and forms an optical image of the subject, two image-capturing devices 6 and 7, such as CCDs, for capturing the optical image formed by the objective lens 5, 2D-image generating sections 8 and 9 for generating 2D images from image information acquired by the image-capturing devices 6 and 7, and a prism-type beam splitter 10 that is disposed between the objective lens 5 and the two image-capturing devices 6 and 7.

Fig. 2 is a diagram showing, in enlarged form, the objective lens 5 and the prism-type beam splitter 10, which are included in the main body 2.

An objective lens that has a diameter of 4 mm and an angle-of-view of 80°, for example, is used as the objective lens 5.

The prism-type beam splitter 10 includes two right-angle prisms (focal-position adjusting sections) 11 and 12 whose inclined faces are joined together and a beam splitter (light splitting section) 13 that is made of a dielectric film provided on a joint surface of the right-angle prisms 11 and 12. The right-angle prisms 11 and 12 are located such that their inclined faces intersect with the optical axis of the objective lens 5 at 45°.

Light entering the front-side first right-angle prism 11 from the objective lens 5 is split into two beams by the beam splitter 13 such that the two beams have almost the same light intensity. One of the two split beams is deflected from the optical axis of the objective lens 5 by 90° and is imaged in the first image-capturing device 6. The other of the two split beams is transmitted through the beam splitter 13, passes through the second right-angle prism 12, and is imaged in the second image-capturing device 7.

Here, the right-angle prisms 11 and 12 give different optical path lengths to the two beams of the light split by the beam splitter 13. For example, as shown in Fig. 2, the sizes of the right-angle prisms 11 and 12 are set such that, when the optical path length of one of the beams of the light is a + b, and the optical path length of the other beam of the light is a + c, b < c is satisfied. Reference symbols a, b, and c all indicate optical path lengths and are defined as the products of the sizes of the right-angle prisms 11 and 12 and the refractive indexes of the right-angle prisms 11 and 12. Thus, the first image-capturing device 6 and the second image-capturing device 7 simultaneously capture optical images of an identical field of view and different focal positions. The difference between the optical path lengths of the two beams of the light can be appropriately designed according to the object distance of the subject determined by the optical design of the objective lens 5 and the main body 2 and the parallax of parallax images to be eventually generated, and is set to 2 mm, for example.

Image information acquired by each of the first image-capturing device 6 and the second image-capturing device 7 is converted into a 2D image by the corresponding 2D-image generating section 8 or 9 and is then sent to the image processing unit 3.

The image processing unit 3 includes a depth-profile generating section 14 that receives two 2D images of different focal positions from the 2D-image generating sections 8 and 9 and generates, through calculation, from two two-dimensional images a depth profile containing information about object distances at individual points on the subject and a parallax-image generating section 15 that generates parallax images by using the depth profile generated by the depth-profile generating section 14.

The depth-profile generating section 14 calculates the object distances between the tip of the objective lens 5 and the individual points on the subject based on the degrees of image blurring of the subject in the two 2D images. In each 2D image, the degree of image blurring becomes larger as the shift length in the direction of the optical axis (in the depth direction) of the objective lens 5 from the focal position is increased. Furthermore, in two 2D images whose focal positions are different from each other, the degree of image blurring of the subject is different from each other. The depth-profile generating section 14 holds a database in which the object distance from the tip of the objective lens 5 to the subject is associated with the degree of image blurring in a 2D image. By referring to the database, the depth-profile generating section 14 calculates an object distance at which the difference between the degrees of blurring in the two 2D images at each point is minimized, as the object distance of the subject at that point.

The parallax-image generating section 15 generates, through calculation, the parallax images by using the depth profile generated by the depth-profile generating section 14. The parallax images are a pair of viewpoint-images of the subject when viewed from two viewpoints corresponding to the right and left eyes of an observer. The parallax-image generating section 15 outputs the generated parallax images to the stereoscopic image display unit 4.

The stereoscopic image display unit 4 creates a stereoscopic image of the subject from the parallax images received from the parallax-image generating section 15 and displays the stereoscopic image.

In this case, according to the stereoscopic endoscope device 1 of this embodiment, the image processing unit 3 receives, from the 2D-image generating sections 8 and 9, two 2D images that are simultaneously acquired by the two image-capturing devices 6 and 7, then successively generates parallax images, and outputs them to the stereoscopic image display unit 4. Specifically, because the time required to generate the parallax images is sufficiently short, the stereoscopic image display unit 4 can display 2D images of the subject that are acquired by the image-capturing devices 6 and 7 consecutively as a moving image, almost in real-time as a stereoscopic moving image.

Next, the parallax images generated by the parallax-image generating section 15 will be described in more detail.

In order to generate the parallax images, as shown in Fig. 3, it is necessary to set the positions of two viewpoints A and B from which a subject X is observed and the directions of the lines of sight along which the subject X is observed from the viewpoints A and B. When the depthwise positions of the viewpoints A and B are equal to the position of the tip of the objective lens 5 (specifically, when the object distance is zero), the positions of the viewpoints A and B and the directions of the lines of sight are geometrically determined by setting a base-line length d, an inward angle θ, and a crossover distance r. The base-line length d is the distance between the viewpoints A and B. The inward angle θ is the angle between two lines of sight that connect the viewpoints A and B and a point of regard O. The crossover distance r is the depthwise distance between the point of regard O, at which the two lines of sight intersect, and the viewpoints A and B.

In this embodiment, the point of regard O is the center of a 2D image and is the point having the same object distance as a far end (point farthest from the tip of the objective lens 5, among points on the subject X) P of the subject X. Specifically, the crossover distance r is the object distance at the far end P of the subject X and is uniquely determined by the subject X. By setting the crossover distance r as the object distance at the far end P, it is possible to eliminate an area that appears in only one of the viewpoint images. The base-line length d and the inward angle θ change interdependently, and therefore, when one of them is determined, the other is also determined. An axis S matches the optical axis of the objective lens 5.

On the other hand, a sense of depth that the observer, who observes a generated stereoscopic image, perceives from the stereoscopic image is determined according to the angle-of-view of the main body 2, the angle-of-view of the stereoscopic image display unit 4, which displays the stereoscopic image, the space between the right and left eyes of the observer, and the angle between the two lines of sight when an identical point of regard is viewed with the right and left eyes (angle-of-convergence). However, all of these conditions have fixed values or preset appropriate values. Therefore, practical parameters for adjusting a sense of depth of the stereoscopic image that is given to the observer are the above-described crossover distance r, base-line length d, and inward angle θ.

Here, the relationship between the base-line length d and a sense of depth of the stereoscopic image perceived by the observer will be described. Figs. 4A, 4B, and 4C show the relationships between the object distance and a reproduction distance when the base-line length d is changed to 5 mm, 3 mm, and 1 mm. The reproduction distance is a depthwise distance of the subject X that the observer perceives from the stereoscopic image displayed on the stereoscopic image display unit 4 and is calculated based on the angle-of-view of the stereoscopic image display unit 4 and the angle-of-convergence of the observer. Graphs shown in Figs. 4A to 4C are made on the assumption that the object distance at the far end P of the subject X is 60 mm, and the crossover distance r is set to 60 mm in the calculation. Furthermore, in these graphs, the calculation is performed by setting the angle-of-view of the stereoscopic image display unit 4 to 40° and the angle-of-convergence of the observer to 5°.

When the base-line length d is set to 5 mm, as shown in Fig. 4A, the object distance and the reproduction distance have a non-linear relationship in which, as the object distance is increased, the variation in the reproduction distance with respect to the variation in the object distance is increased, and the graph is formed of a curve that is convex downward. In this relationship, a so-called puppet theater effect, in which a near subject appears to protrude and be small compared with a far subject, occurs in a stereoscopic image. Specifically, in the stereoscopic image, a near subject is displayed as if it is located nearer than its actual location, and a far subject is displayed as if it is located farther than its actual location. Therefore, it is difficult for the observer to understand the accurate stereoscopic shape of the subject from the stereoscopic image. Furthermore, such a stereoscopic image in which a sense of depth is excessively emphasized gives the observer a feeling of intense fatigue.

When the base-line length d is set to 3 mm, as shown in Fig. 4B, the object distance and the reproduction distance have a substantially linear relationship, and a sense of depth of the subject X is accurately reproduced in a stereoscopic image. In this relationship, a sense of depth perceived when the subject X is actually viewed with the naked eyes conforms with a sense of depth of the subject in the stereoscopic image. Therefore, the observer can easily understand, from the stereoscopic image, an accurate depthwise positional relationship between the tissue, which is the subject X, and a treatment tool.

When the base-line length d is set to 1 mm, as shown in Fig. 4C, the object distance and the reproduction distance have a non-linear relationship in which, as the object distance is increased, the variation in the reproduction distance with respect to the variation in the object distance is reduced, and the graph is formed of a curve that is convex upward. Then, a so-called cardboard effect, in which the subject is compressed in the depth direction, occurs in the stereoscopic image. Such a relationship is suitable for a case in which a subject whose shape changes by a large amount in the depth direction is observed. Specifically, the observer needs to change his/her convergence to view different-depth positions in the stereoscopic image, thus getting easily tired as the difference in depth is increased. In contrast to this, in the stereoscopic image in which the subject is compressed in the depth direction, because the accommodation range for the convergence is small, a feeling of fatigue given to the observer can be reduced.

The parallax-image generating section 15 holds a table recording combinations of the crossover distance r and the base-line length d that cause the object distance and the reproduction distance to have a substantially linear relationship, as shown in Fig. 4B. The parallax-image generating section 15 extracts the maximum value of the object distance from the depth profile, sets the extracted maximum value of the object distance as the crossover distance r, and sets the base-line length d associated with the set crossover distance r by referring to the table. Then, parallax images are generated by using the set crossover distance r and base-line length d.

A stereoscopic image created from the parallax images generated in this way gives the observer a sense of depth of the subject conforming with a sense of depth of the actual subject X. Therefore, an advantage is afforded in that the observer can always understand an accurate depthwise position of the subject X from the stereoscopic image.

Furthermore, in the case of a twin-lens stereoscopic endoscope that acquires parallax images of a subject by using two objective lenses, because the distance between the optical axes of the right and left objective lenses, which indicates the base-line length, has a lower limit, the base-line length cannot be reduced sufficiently. For example, when two objective lenses having a diameter of 4 mm, which is the same diameter as that of the objective lens used in this embodiment, are arranged side by side, the lower limit of the base-line length exceeds 4 mm, thereby making it impossible to generate parallax images with the base-line length set to 3 mm or 1 mm.

Since the subject X to be observed using the endoscope is small, with a size from several mm to 10 mm, the base-line length needs to be reduced as well in order to reproduce, in the stereoscopic image, a sense of depth of the subject equivalent to a sense of depth of the actual subject X. However, in a stereoscopic image acquired by the twin-lens stereoscopic endoscope, the sense of depth is excessively emphasized because the base-line length is too large for the size of the subject. In contrast to this, according to this embodiment, it is possible to set the base-line length d for the parallax images to a value equal to or smaller than the diameter of the objective lens 5 and to generate a stereoscopic image having an appropriate sense of depth.

Furthermore, the depth profile can also be generated by using a single 2D image. In that case, however, because it is impossible to determine whether the variation in luminance value in the 2D image is caused by the stereoscopic shape of the image or by image blurring, an error can possibly be caused in a calculated object distance. For example, a convex shape is obtained as a concave shape through calculation, thereby making it impossible to reproduce the accurate stereoscopic shape of the subject, in some cases. In contrast to this, according to this embodiment, two 2D images are used, and an object distance is calculated such that a difference between the degrees of blurring in the two 2D images is minimized, thereby making it possible to accurately calculate the object distance. Thus, it is possible to generate a stereoscopic image in which the stereoscopic shape of the subject is accurately reproduced.

In this embodiment, the parallax-image generating section 15 sets the crossover distance r and the base-line length d as parameters; however, instead of this, the crossover distance r and the inward angle θ may be set. As described above, the base-line length d and the inward angle θ are values that change interdependently, and therefore, even when the inward angle θ is changed instead of the base-line length d, the object distance and the reproduction distance have the relationships shown in Figs. 4A to 4C. Therefore, the parallax-image generating section 15 may adopt, instead of the base-line length d, the inward angle θ as a parameter set to generate parallax images.

Furthermore, in this embodiment, when generating the parallax images, the parallax-image generating section 15 sets the crossover distance r as the object distance at the far end P of the subject X; however, another value may be adopted as the crossover distance r. In that case, it is preferred that the crossover distance r be set to the distance between two focal positions of optical images to be acquired by the image-capturing devices 6 and 7.

Furthermore, in this embodiment, the parallax-image generating section 15 may set a plurality of base-line lengths d and generate a plurality of parallax images of different base-line lengths d from an identical 2D image. For example, the parallax-image generating section 15 may be configured such that the parallax-image generating section 15 has a first mode in which parallax images are generated with the base-line length d set to 1 mm and a second mode in which parallax images are generated with the base-line length d set to 3 mm and such that the observer can select to output parallax images generated in either mode to the stereoscopic image display unit 4.

In order to observe the whole of the subject X with a downward view, the observer selects the first mode, thereby making it possible to observe the stereoscopic image while reducing the burden on the eyes, even if the subject has depth. On the other hand, in order to observe a part of the subject in detail, the observer selects the second mode, thereby making it possible to accurately understand the stereoscopic shape of the subject X from the stereoscopic image and to accurately perform treatment on the subject, for example.

Furthermore, the parallax-image generating section 15 may switch between the first mode and the second mode based on information about object distances contained in the depth profile. For example, if the difference between the maximum value and the minimum value of the object distances is equal to or larger than a predetermined threshold, the first mode may be selected, and, if the difference therebetween is smaller than the predetermined threshold, the second mode may be selected.

Furthermore, the stereoscopic image display unit 4 may display a pair of viewpoint-images, which are parallax images, displaced in the horizontal direction such that the lines of sight from the right and left eyes of the observer do not intersect at a point and may adjust a sense of depth perceived by the observer by adjusting the distance between the right and left viewpoint images.

When parallax images are stereoscopically viewed, the slope of the curve showing the relationship between the object distance and the reproduction distance (specifically, the position at which curves intersect) is changed according to the angle-of-convergence of the observer. Specifically, the angle-of-convergence of the observer can be adjusted by changing the space between the right and left viewpoint images, and a sense of depth of the subject perceived by the observer from the stereoscopic image can be reduced by increasing the angle-of-convergence.

Furthermore, the configurations of the light splitting section, the focal-position adjusting sections, and the image-capturing devices, which are described in this embodiment, are merely examples, and they are not limited to these configurations.

Figs. 5 to 10 show modifications of the light splitting section, the focal-position adjusting sections, and the image-capturing devices.

In Fig. 5, the focal-position adjusting sections are formed of two joined prisms 111a and 111b, and the light splitting section is formed of a beam splitter 131 that is provided on the joint surface of the prisms 111a and 111b. In this configuration, two beams of light split by the beam splitter 131 are output from the two prisms 111a and 111b in parallel to each other and are captured at different areas of a common image-capturing device 61. By doing so, because only the single image-capturing device 61 suffices, it is possible to achieve a simpler configuration of an end portion of the main body 2 and a reduction in size of the end portion.

In Fig. 6, the focal-position adjusting sections are formed of two joined prisms 112a and 112b, and the light splitting section is formed of a polarization beam splitter 132a that is provided on the joint surface of the prisms 112a and 112b, a retarder 16 that gives a phase difference to a part of light
deflected by the polarization beam splitter 132a, and a mirror 17 that returns light entering the retarder 16 toward the opposite side. By doing so, it is possible to more freely set the difference between the optical path lengths of two beams of light split by the polarization beam splitter 132a.

In Fig. 7, the focal-position adjusting sections are formed of four prisms 113a, 113b, 113c, and 113d that are joined with one another, and the light splitting section is formed of two beam splitters 133a and 133b provided on the joint surfaces of the four prisms 113a, 113b, 113c, and 113d. The four prisms 113a, 113b, 113c, and 113d are joined such that the joint surfaces thereof form two planes that intersect perpendicularly to each other, and light from the objective lens 5 (not shown) is split into three beams by the two beam splitters 133a and 133b.

The three split beams of light are captured by different image-capturing devices 63a, 63b, and 63c. By doing so, the depth-profile generating section 14 generates a depth profile from three 2D images of different focal positions. Therefore, it is possible to generate a more accurate depth profile for a subject whose shape changes by a large amount in the depth direction and to create a stereoscopic image in which the stereoscopic shape of the subject is more accurately reproduced.

Fig. 8 shows a modification in which a prism 112c, a beam splitter 132b, and an image-capturing device 62b are added to the configuration shown in Fig. 6, and light entering from the objective lens 5 (not shown) is split into three beams, thereby making it possible to acquire three 2D images of different focal positions.

Fig. 9 shows a modification in which light from the objective lens 5 (not shown) is split into three by prisms 114a, 114b, and 114c alone, and the split beams of light are captured by image-capturing devices 64a, 64b, and 64c. Specifically, the prisms 114a, 114b, and 114c constitute the light splitting section and the focal-position adjusting sections.

Furthermore, in this embodiment, the image-capturing devices 6 and 7 may each have, on its imaging plane, an imaging-state detecting section that detects the light imaging state on the imaging plane by a phase difference method. In conventional technologies, the phase difference between images formed by light flux passing through different pupil areas is detected, thereby detecting the imaging state.

As an imaging-state detecting section 18, as shown in Fig. 10, an optical filter described in Japanese Unexamined Patent Application, Publication No. 2012-22147 is preferably adopted. Of light from the objective lens 5, this optical filter allows light flux passing through one pupil area to enter a certain row of pixels of the image-capturing device 6 or 7 and allows light flux passing through another pupil area to enter another row of pixels that is provided in parallel to the certain row of pixels and in the vicinity of the certain row of pixels. In the figure, reference symbols 18h and 18v denote viewing-angle pupil control elements, and reference symbol 18a denotes a transparent member.

The imaging-state detecting section may be configured such that a light blocking mask allows only part of light passing through the pupil area to pass therethrough. Alternatively, the imaging-state detecting section may be configured such that the position of a microlens is adjusted, thereby allowing light passing through a different pupil area to enter a row of pixels.

By providing the imaging-state detecting section in this way, distance information of the detecting section can be accurately measured, and the accuracy of distance estimation of the whole of the imaging plane can be further enhanced by adding this accurate distance information.

### {Reference Signs List}

- 1: stereoscopic endoscope device
- 2: endoscope main body
- 3: image processing unit
- 4: stereoscopic image display unit (display unit)
- 5: objective lens
- 6, 7: image-capturing devices
- 8, 9: 2D-image generating sections
- 10: prism-type beam splitter
- 11, 12: right-angle prisms (focal-position adjusting sections)
- 13: beam splitter (light splitting section)
- 14: depth-profile generating section (calculation section)
- 15: parallax-image generating section
- 16: retarder
- 17: mirror
- 18: imaging-state detecting section
- A, B: viewpoints
- X: subject
- d: base-line length
- r: crossover distance
- O: point of regard
- S: axis
- θ: inward angle

## Claims

1. A stereoscopic endoscope device (1) comprising:
a single objective lens (5) adapted to collect light from a subject (X) and to form an image of the light;
a light splitting section (13) adapted to split the light collected by the objective lens (5) into two or more beams,
focal-position adjusting sections (11, 12) adapted to give optical path lengths different from each other to the two or more beams of the light split by the light splitting section (13),
two image-capturing devices (6, 7) that are disposed at imaging positions of the beams of the light split by the light splitting section (13) and adapted to simultaneously capture optical images of the subject (X);
a calculation section (14) configured to calculate an object distance between each point on the subject (X) and the objective lens (5), from two 2D images of the subject (X) that are simultaneously acquired by the two image-capturing devices (6, 7) and whose focal positions are different from each other; and
a parallax-image generating section (15) configured to generate a plurality of viewpoint-images of the subject (X) when observed from a plurality of viewpoints (A, B), by using the object distance calculated by the calculation section (14), wherein the calculation section (14) is configured to calculate the object distance between the tip of the objective lens (5) and each point on the subject (X) based on the degrees of image blurring of the subject (X) in the two 2D images, the degree of image blurring in each 2D image becoming larger as the shift length in the direction of the optical axis in the depth direction of the objective lens (5) from the focal position is increased,
**characterized in that** the calculation section (14) has a database in which the object distance from the tip of the objective lens (5) to the subject (X) is associated with the degree of image blurring in a 2D image, such that, by referring to the database, the calculation section (14) configured to calculate an object distance at which the difference between the degrees of blurring in the two 2D images at each point is minimized, as the object distance of the subject (X) at that point.

2. The stereoscopic endoscope device (1) according to claim 1, wherein the parallax-image generating section (15) is configured to set a space between the plurality of viewpoints (A, B) to a distance smaller than a diameter of the objective lens (5).

3. The stereoscopic endoscope device (1) according to claim 1 or 2, comprising a display unit (4) configured to create a stereoscopic image from the plurality of viewpoint-images generated by the parallax-image generating section (15) and displays the stereoscopic image,
wherein the parallax-image generating section (15) is configured to generate parallax images in which the object distance and a depthwise reproduction distance reproduced in the stereoscopic image displayed in the display unit (4) have a substantially linear relationship.

4. The stereoscopic endoscope device (1) according to claim 1 or 2, comprising a display unit (4) configured to create a stereoscopic image from the plurality of viewpoint-images generated by the parallax-image generating section (15) and to display the stereoscopic image,
wherein the parallax-image generating section (15) is configured to generate parallax images in which the object distance and a depthwise reproduction distance reproduced in the stereoscopic image displayed in the display unit (4) have a non-linear relationship in which a variation in the reproduction distance with respect to a variation in the object distance is convex upward.

5. The stereoscopic endoscope device (1) according to any one of claims 1 to 4, wherein the parallax-image generating section (15) is configured to set a space between the plurality of viewpoints (A, B) to 5 mm or less.

6. The stereoscopic endoscope device (1) according to any one of claims 1 to 5, wherein the parallax-image generating section (15) is configured to set a space between the plurality of viewpoints (A, B) to a plurality of distances and generates a plurality of parallax images.

7. The stereoscopic endoscope device (1) according to any one of claims 1 to 6,
wherein the light splitting section (13) is adapted to output the two or more split beams of the light almost parallel to each other; and
an imaging plane of the two image-capturing devices (6, 7) is divided into two or more areas, and the two or more beams of the light output from the light splitting section (13) are captured in different two or more areas.

8. The stereoscopic endoscope device (1) according to any one of claims 1 to 7,
wherein the focal-position adjusting sections (11, 12) comprise two prisms that are joined together and are disposed such that a joint surface of the two prisms intersects with an optical axis (S) of the objective lens (5); and
the light splitting section (13) comprises a beam splitter that is provided on the joint surface and that allows a part of light entering one of the prisms from the objective lens (5) to be transmitted through the other prism and the other part of the light to be deflected in a direction intersecting the optical axis (S).

9. The stereoscopic endoscope device (1) according to any one of claims 1 to 8, wherein the light splitting section (13) has an outer diameter smaller than outer diameters of the two image-capturing devices (6, 7).

10. The stereoscopic endoscope device (1) according to any one of claims 1 to 9, wherein the parallax-image generating section (15) is configured to generate parallax images such that an intersection of virtual lines of sight for observing the subject (X) from viewpoints (A, B) is located between focal positions of the two or more beams of the light to be captured by the two image-capturing devices (6, 7).

11. The stereoscopic endoscope device (1) according to any one of claims 1 to 10, wherein each of the two image-capturing devices (6, 7) comprises an imaging-state detecting section (18) configured to detect an imaging state of the light to be captured, by a phase difference method.

## Patentansprüche

1. Stereoskopische Endoskopvorrichtung (1), umfassend:
eine einzelne Objektivlinse (5), die dazu angepasst ist, Licht von einem Subjekt (X) zu sammeln und ein Bild von dem Licht zu erzeugen;
einen Lichtteilungsabschnitt (13), der dazu angepasst ist, das von der Objektivlinse (5) gesammelte Licht in zwei oder mehrere Strahlen zu teilen,
Fokuslageneinstellabschnitte (11, 12), die dazu angepasst sind, den zwei oder mehreren Strahlen des von dem Lichtteilungsabschnitt (13) geteilten Lichts optische Weglängen, die sich voneinander unterscheiden, zu verleihen,
zwei Bilderfassungsvorrichtungen (6, 7), die an Abbildungspositionen der Strahlen des von dem Lichtteilungsabschnitt (13) geteilten Lichts angeordnet und dazu angepasst sind, gleichzeitig optische Bilder des Subjekts (X) zu erfassen;
einen Berechnungsabschnitt (14), der dazu ausgelegt ist, einen Objektabstand zwischen jedem Punkt auf dem Subjekt (X) aus zwei 2D-Bildern des Subjekts (X) zu berechnen, die gleichzeitig von den zwei Bilderfassungsvorrichtungen (6, 7) aufgenommen wurden, und deren Fokuslagen sich voneinander unterscheiden; und
einen Parallaxbilderzeugungsabschnitt (15), der dazu ausgelegt ist, eine Mehrzahl von Standortbildern des Subjekts (X), wenn aus einer Mehrzahl von Standorten (A, B) beobachtet, mithilfe des von dem Berechnungsabschnitt (14) berechneten Objektabstands zu erzeugen,
wobei der Berechnungsabschnitt (14) ferner dazu ausgelegt ist, den Objektabstand zwischen der Spitze der Objektivlinse (5) und jedem Punkt auf dem Subjekt (X) basierend auf den Graden der Bildunschärfe des Subjekts (X) in den zwei 2D-Bildern zu berechnen, wobei der Grad der Bildunschärfe in jedem 2D-Bild größer wird, wenn die Verschiebungslänge in der Richtung der optischen Achse in der Tiefenrichtung der Objektivlinse (5) aus der Fokuslage erhöht wird,
**dadurch gekennzeichnet, dass** der Berechnungsabschnitt (14) eine Datenbank aufweist, in der der Objektabstand von der Spitze der Objektivlinse (5) bis zu dem Subjekt (X) mit dem Grad der Bildunschärfe in einem 2D-Bild verknüpft ist, sodass durch Bezugnahme auf die Datenbank der Berechnungsabschnitt (14) dazu ausgelegt ist, einen Objektabstand, bei dem die Differenz zwischen den Graden der Unschärfe in den zwei 2D-Bildern an jedem Punkt minimiert wird, als Objektabstand des Subjekts (X) an diesem Punkt zu berechnen.

2. Stereoskopische Endoskopvorrichtung (1) nach Anspruch 1, wobei der Parallaxbilderzeugungsabschnitt (15) dazu ausgelegt ist, einen Zwischenraum zwischen der Mehrzahl von Standorten (A, B) auf einen Abstand einzustellen, der kleiner ist als ein Durchmesser der Objektivlinse (5).

3. Stereoskopische Endoskopvorrichtung (1) nach Anspruch 1 oder 2, die eine Anzeigeeinheit (4) umfasst, die dazu ausgelegt ist, ein Stereobild aus der Mehrzahl von Standortbildern zu erstellen, die von dem Parallaxbilderzeugungsabschnitt (15) erzeugt wurden, und das Stereobild anzuzeigen,
wobei der Parallaxbilderzeugungsabschnitt (15) dazu ausgelegt ist, Parallaxbilder zu erzeugen, in welchen der Objektabstand und ein Tiefenwiedergabeabstand, der in dem Stereobild, das in der Anzeigeeinheit (4) angezeigt wird, wiedergegeben wurde, eine im Wesentliche lineare Beziehung aufweisen.

4. Stereoskopische Endoskopvorrichtung (1) nach Anspruch 1 oder 2, die eine Anzeigeeinheit (4) umfasst, die dazu ausgelegt ist, ein Stereobild aus der Mehrzahl von Standortbildern zu erstellen, die von dem Parallaxbilderzeugungsabschnitt (15) erzeugt wurden, und das Stereobild anzuzeigen,
wobei der Parallaxbilderzeugungsabschnitt (15) dazu ausgelegt ist, Parallaxbilder zu erzeugen, in welchen der Objektabstand und ein Tiefenwiedergabeabstand, der in dem Stereobild, das in der Anzeigeeinheit (4) angezeigt wird, wiedergegeben wurde, eine nichtlineare Beziehung aufweisen, in der eine Variation in dem Wiedergabeabstand mit Bezug auf eine Variation in dem Objektabstand konvex ansteigend ist.

5. Stereoskopische Endoskopvorrichtung (1) nach einem der Ansprüche 1 bis 4, wobei der Parallaxbilderzeugungsabschnitt (15) dazu ausgelegt ist, einen Zwischenraum zwischen der Mehrzahl von Standorten (A, B) auf 5 mm oder weniger einzustellen.

6. Stereoskopische Endoskopvorrichtung (1) nach einem der Ansprüche 1 bis 5, wobei der Parallaxbilderzeugungsabschnitt (15) dazu ausgelegt ist, einen Zwischenraum zwischen der Mehrzahl von Standorten (A, B) auf eine Mehrzahl von Abständen einzustellen, und eine Mehrzahl von Parallaxbildern erzeugt.

7. Stereoskopische Endoskopvorrichtung (1) nach einem der Ansprüche 1 bis 6,
wobei der Lichtteilungsabschnitt (13) dazu angepasst ist, die zwei oder mehreren geteilten Strahlen des Lichts nahezu parallel zueinander auszugeben; und
eine Bildebene der zwei Bilderfassungsvorrichtungen (6, 7) in zwei oder mehrere Flächen unterteilt ist, und die zwei oder mehreren Strahlen des aus dem Lichtteilungsabschnitt (13) ausgegebenen Lichts in anderen zwei oder mehreren Flächen erfasst werden.

8. Stereoskopische Endoskopvorrichtung (1) nach einem der Ansprüche 1 bis 7,
wobei die Fokuslageneinstellabschnitte (11, 12) zwei Prismen umfassen, die miteinander verbunden und derart angeordnet sind, dass eine Anschlussfläche der zwei Prismen eine optische Achse (S) der Objektivlinse (5) schneidet; und
der Lichtteilungsabschnitt (13) einen Strahlteiler umfasst, der an der Anschlussfläche vorgesehen ist und der ermöglicht, dass ein Teil des Lichts, das in eins der Prismen der Objektivlinse (5) eintritt, durch das andere Prisma gesendet wird, und der andere Teil des Lichts in eine Richtung, die die optische Achse (S) schneidet, abgelenkt wird.

9. Stereoskopische Endoskopvorrichtung (1) nach einem der Ansprüche 1 bis 8, wobei der Lichtteilungsabschnitt (13) einen Außendurchmesser aufweist, der kleiner ist als die Außendurchmesser der zwei Bilderfassungsvorrichtungen (6, 7).

10. Stereoskopische Endoskopvorrichtung (1) nach einem der Ansprüche 1 bis 9, wobei der Parallaxbilderzeugungsabschnitt (15) dazu ausgelegt ist, Parallaxbilder zu erzeugen, sodass sich ein Schnittpunkt virtueller Sichtlinien zum Beobachten des Subjekts (X) von Standorten (A, B) zwischen Fokuslagen der zwei oder mehreren Strahlen des von den zwei Bilderfassungsvorrichtungen (6, 7) zu erfassenden Lichts befindet.

11. Stereoskopische Endoskopvorrichtung (1) nach einem der Ansprüche 1 bis 10, wobei jede der zwei Bilderfassungsvorrichtungen (6, 7) einen Bildzustandserfassungsabschnitt (18) umfasst, der dazu ausgelegt ist, einen Bildzustand des zu erfassenden Lichts durch ein Phasendifferenzverfahren zu erfassen.

## Revendications

1. Dispositif d'endoscope stéréoscopique (1) comprenant :
un unique objectif (5) adapté pour collecter de la lumière provenant d'un sujet (X) et former une image de la lumière ;
une section de division de lumière (13) adaptée pour diviser la lumière collectée par l'objectif (5) en deux faisceaux ou plus,
des sections d'ajustement de position focale (11, 12) adaptées pour fournir des longueurs de trajet optique différentes l'une de l'autre aux au moins deux faisceaux de la lumière divisée par la section de division de lumière (13),
deux dispositifs de capture d'image (6, 7) qui sont disposés dans des positions d'imagerie des faisceaux de la lumière divisée par la section de division de lumière (13) et adaptés pour capturer simultanément des images optiques du sujet (X) ;
une section de calcul (14) configurée pour calculer une distance d'objet entre chaque point sur le sujet (X) et l'objectif (5), à partir de deux images 2D du sujet (X) qui sont acquises simultanément par les deux dispositifs de capture d'image (6, 7), et dont les positions focales sont différentes l'une de l'autre ; et
une section de génération d'image de parallaxe (15) configurée pour générer une pluralité d'images de point de vue du sujet (X) lorsqu'observé depuis une pluralité de points de vue (A, B), en utilisant la distance d'objet calculée par la section de calcul (14),
la section de calcul (14) étant configurée pour calculer la distance d'objet entre la pointe de l'objectif (5) et chaque point sur le sujet (X) sur la base des degrés de flou d'image du sujet (X) dans les deux images 2D, le degré de flou d'image dans chaque image 2D augmentant à mesure que la longueur de décalage dans la direction de l'axe optique dans la direction de profondeur de l'objectif (5) depuis la position focale augmente,
**caractérisé par le fait que** la section de calcul (14) a une base de données dans laquelle la distance d'objet de la pointe de l'objectif (5) au sujet (X) est associée au degré de flou d'image dans une image 2D de telle sorte que, en se référant à la base de données, la section de calcul (14) est configurée pour calculer une distance d'objet à laquelle la différence entre les degrés de flou dans les deux images 2D au niveau de chaque point est rendue minimale, en tant que distance d'objet du sujet (X) au niveau de ce point.

2. Dispositif d'endoscope stéréoscopique (1) selon la revendication 1, dans lequel la section de génération d'image de parallaxe (15) est configurée pour définir un espace entre la pluralité de points de vue (A, B) à une distance inférieure à un diamètre de l'objectif (5).

3. Dispositif d'endoscope stéréoscopique (1) selon la revendication 1 ou 2, comprenant une unité d'affichage (4) configurée pour créer une image stéréoscopique à partir de la pluralité d'images de point de vue générées par la section de génération d'image de parallaxe (15) et affiche l'image stéréoscopique,
la section de génération d'image de parallaxe (15) étant configurée pour générer des images de parallaxe dans lesquelles la distance d'objet et une distance de reproduction de profondeur reproduite dans l'image stéréoscopique affichée dans l'unité d'affichage (4) ont une relation sensiblement linéaire.

4. Dispositif d'endoscope stéréoscopique (1) selon la revendication 1 ou 2, comprenant une unité d'affichage (4) configurée pour créer une image stéréoscopique à partir de la pluralité d'images de point de vue générées par la section de génération d'image de parallaxe (15) et afficher l'image stéréoscopique,
la section de génération d'image de parallaxe (15) étant configurée pour générer des images de parallaxe dans lesquelles la distance d'objet et une distance de reproduction de profondeur reproduite dans l'image stéréoscopique affichée dans l'unité d'affichage (4) ont une relation non-linéaire dans laquelle une variation de la distance de reproduction par rapport à une variation de la distance d'objet est convexe vers le haut.

5. Dispositif d'endoscope stéréoscopique (1) selon l'une quelconque des revendications 1 à 4, dans lequel la section de génération d'image de parallaxe (15) est configurée pour définir un espace entre la pluralité de points de vue (A, B) à 5 mm ou moins.

6. Dispositif d'endoscope stéréoscopique (1) selon l'une quelconque des revendications 1 à 5, dans lequel la section de génération d'image de parallaxe (15) est configurée pour définir un espace entre la pluralité de points de vue (A, B) à une pluralité de distances et génère une pluralité d'images de parallaxe.

7. Dispositif d'endoscope stéréoscopique (1) selon l'une quelconque des revendications 1 à 6, dans lequel la section de division de lumière (13) est adaptée pour délivrer en sortie les au moins deux faisceaux de la lumière divisée, presque parallèles l'un à l'autre ; et
un plan d'imagerie des deux dispositifs de capture d'image (6, 7) est divisé en deux zones ou plus, et les au moins deux faisceaux de la lumière délivrés en sortie par la section de division de lumière (13) sont capturés dans au moins deux zones différentes.

8. Dispositif d'endoscope stéréoscopique (1) selon l'une quelconque des revendications 1 à 7,
dans lequel les sections d'ajustement de position focale (11, 12) comprennent deux prismes qui sont reliés ensemble et son disposés de telle sorte qu'une surface de liaison des deux prismes coupe un axe optique (S) de l'objectif (5) ; et
la section de division de lumière (13) comprend un diviseur de faisceau qui est disposé sur la surface de liaison et qui permet à une partie de la lumière entrant dans l'un des prismes depuis l'objectif (5) d'être transmise à travers l'autre prisme et permet à l'autre partie de la lumière d'être déviée dans une direction coupant l'axe optique (S).

9. Dispositif d'endoscope stéréoscopique (1) selon l'une quelconque des revendications 1 à 8, dans lequel la section de division de lumière (13) a un diamètre externe plus petit que des diamètres externes des deux dispositifs de capture d'image (6, 7).

10. Dispositif d'endoscope stéréoscopique (1) selon l'une quelconque des revendications 1 à 9, dans lequel la section de génération d'image de parallaxe (15) est configurée pour générer des images de parallaxe de telle sorte qu'une intersection de lignes de vision virtuelles pour observer le sujet (X) à partir de points de vue (A, B) est située entre les positions focales des au moins deux faisceaux de la lumière à capturer par les deux dispositifs de capture d'image (6, 7).

11. Dispositif d'endoscope stéréoscopique (1) selon l'une quelconque des revendications 1 à 10, dans lequel chacun des deux dispositifs de capture d'image (6, 7) comprend une section de détection d'état d'imagerie (18) configurée pour détecter un état d'imagerie de la lumière à capturer, par une méthode de différence de phase.
